# EUROPÄISCHE PATENTANMELDUNG

(11) **EP 1 186 314 A2**
(43) Veröffentlichungstag der Anmeldung: **13.03.2002**
(21) Anmeldenummer: 01121510.0
(22) Anmeldetag: 08.09.2001
(51) Int. Cl.: A61M 16/06

(54) **Individualmaske und Verfahren zum Herstellen einer Individualmaske**

(30) Priorität: 11.09.2000 DE 10044977; 24.03.2001 DE 10114653
(71) Anmelder: Helmut Eglseder Dental GmbH, 93073 Neutraubling (DE)
(72) Erfinder: Helmut Eglseder Dental GmbH, 93073 Neutraubling (DE)
(74) Vertreter: Wasmeier, Alfons, Dipl.-Ing.

(57) **Zusammenfassung**

Zum Abformen und Anfertigen einer nasalen Maske für Überdruckbeatmung wird auf die abzudeckende Gesichtspartie eine Trennschicht aufgebracht, selbsthärtendes Abformmaterial gemischt und zu einer zähflüssigen Masse angerührt, die Abformmasse drucklos auf die abzudeckende Gesichtspartie aufgebracht, die Abformung nach dem Abbinden abgenommen und von dem Negativ ein Positiv als Arbeitsmodell erstellt, sowie von dem Positiv eine individuelle Beatmungsmaske hergestellt. Zusätzlich bzw bei Bedarf können die empfindlichen Gesichtspartien mit weichbleibendem Kunststoff oder einem Heißpolymerisator unterfüttert werden.

## Beschreibung

Die Erfindung betrifft ein Verfahren nach dem Oberbegriff des Anspruches 1 und eine nach diesem Verfahren hergestellte Maske nach dem Oberbegriff des Anspruches 2.

Für die Durchführung einer nasalen Überdruck-Beatmung oder einer intermittierenden Selbstbeatmung werden weltweit konfektionierte Masken hergestellt, die in mehreren Materialien, Formen und Größen auf Lager gehalten werden und die mehr oder weniger gut der Gesichtsform den jeweiligen Gesichtsformen der zu beatmenden Patienten entsprechen, da individuelle Anpassungen an die höchst unterschiedlichen Gesichtsformen der Patienten nicht berücksichtigt werden können.

Die Herstellung konfektionierter Masken erfolgt maschinell. In Spritzgusstechnikverfahren wird ein Maskengrundkörper hergestellt, welcher je nach Maskenart mit Gummi oder Silikonmaterial umrandet wird. Die Maske wird aufgrund des verwendeten Materials für den Maskengrundkörper (Kunststoff) sowie der Umrandung (Silikonpolster oder Gummi) relativ schwer und unbeweglich. Es gibt nur wenige Größen unter allen Maskentypen. Durch die Faktoren Gewicht, Unbeweglichkeit und mangelnde Größenauswahl der Masken ergeben sich häufig Druckstellen an den nicht individuell angepassten Kontaktstellen des Gesichtes.

Individualmasken sollten sämtlichen Kriterien entsprechen, die dem Patienten die tägliche Anwendung erleichtern. Derartige Individualmasken haben jedoch auf allen Ebenen nur einen Nachteil. Sie werden nach komplizierten technischen Verfahren hergestellt und sind somit mit einem hohen finanziellen Kostenfaktor verbunden, da eine ausschließlich handwerkliche Fertigung vorliegt. Hierbei steht die jeweils individuelle Anfertigung eines Kunststoff-Prothesenkörpers im Vordergrund.

Aufgabe der Erfindung ist, für den angegebenen Einsatzbereich Individual-Masken herzustellen und vor allem Nachteile konfektionierter Masken und der bisher gefertigten Individualmasken aufgrund der oben beschriebenen drucklosen Abformung und Herstellungstechnik von vorneherein Druckstellen und Undichtigkeiten zu vermeiden. Es kann somit jede Gesichts- bzw. Nasenform, Nasengröße sowie jede Abnormität des Gesichtes (z.B. Narben, Verbrennungen, Glasaugeträger, Prothesenträger usw) berücksichtigt werden. Des weiteren ist Aufgabe der Erfindung, eine nach diesem Verfahren hergestellte Maske anzugeben, die eine für den Patienten einfach zu handhabende, passgenaue und auf Dauer kostengünstigere Anwendung (für Krankenkassen) einer nasalen Beatmungsmaske darstellt, da diese Individualmaske jederzeit bei Gewichtsab- oder -zunahme indirekt unterfüttert werden kann bzw. bei Brüchen, Sprüngen usw. jederzeit mit dem Ausgangsmaterial repariert werden kann. Diese Materialien sind seit Jahren in der Dentaltechnik im Einsatz und entsprechen dem Medizinproduktegesetz.

Das Verfahren zum Herstellen derartiger Masken wird nach den Merkmalen des Kennzeichens des Anspruches 1 durchgeführt. Eine Maske nach der Erfindung ist durch die Merkmale des Kennzeichens des Anspruches 2 bestimmt.

Nach dem erfindungsgemäßen Verfahren wird das Herstellen der Maske durch drucklose Abformung vorgenommen. Dabei wird Abformgips oder Silikon mit Wasser bzw. den Herstellerangaben entsprechend angerührt, der Patient wird in horizontaler Lage mit Vaseline oder dergl. Trennmaterial an den abzuformenden Partien, z.B. dem Gesicht, gegen das aufzutragende Abformmaterial isoliert. Die Öffnungsstellen der abzuformenden Partie, z.B. im Falle des Gesichtes die Augen, der Mund und die Nasenlöcher, werden dabei z.B. mit Zellstoff abgedeckt. Das Abformmaterial wird in zähflüssiger Konsistenz auf die abzuformende Partie in einer Stärke von 3 - 5 cm aufgetragen, z.B. aufgelegt, so dass das Abformmaterial sich genau der abzuformenden Partie, insbesondere des Gesichtes und der Gesichtszüge anpasst.

Als Abformmaterial wird vorzugsweise Abformgips oder Silikon verwendet. Sobald das Abformmaterial abgebunden hat, wird das den Gesichtszügen genau angepasste Negativ von einigen cm Dicke abgenommen und anschließend ein Positiv erstellt. Anschließend wird von diesem Positiv nach genauen Angaben des behandelnden Arztes eine Individual-Maske hergestellt, die aus Kunststoff, nämlich einem Kaltpolymerisat besteht. Ein Kaltpolymersisat-Kunststoff wird, ähnlich wie in der Dental-Technik verwendet, z.B. in der Kieferorthopädie oder bei herausnehmbarem Zahnersatz, nach Herstel-lerangaben angerührt und auf das isolierte Arbeitsmodell aufgetragen. Nach Aushärten unter Druck in einem speziell dafür vorgesehenem Drucktopf erfolgt eine anschließen-de Bearbeitung und Reduzierung auf eine gleichmäßige Wandstärke von 2 - 3 mm. Nachdem der gesamte Kunststoffkörper, nämlich die individuelle Beatmungsmaske, fertig ausgearbeitet ist, werden die Halteelemente, deren Anzahl dem Bedarf angepasst ist, zur Fixierung der Maske am Kopf eingesetzt. Der konfektionierte Adapter kann vor der Herstellung des Maskenkörpers oder auch anschließend eingearbeitet werden.

Falls erforderlich, wird an der Maske, die aus Kaltpolymerisat besteht, eine Nachbearbeitung vorgenommen, um individuelle Stellen zu berücksichtigen und anzupassen, erfolgt je nach Bedarf eine weichbleibende Unterfütterung.

Mit der erfindungsgemäßen Methode wird eine drucklos abgeformte Maske in einem einzigen Arbeitsgang hergestellt, während bei unter Druck hergestellten mehrlagigen Masken eine Mehrzahl von Arbeitsgängen und für die Herstellung derartiger Druck-Masken ein Zeitraum von bis zu 14 Tagen erforderlich ist. Die drucklose Abformung ergibt somit einen erheblichen Zeitgewinn bei der Herstellung der Maske, ferner aber auch eine wesentlich bessere Anpassung der Maske an die Gesichtform als bei der Druck-Abformung, so dass mit dem erfindungsgemäßen Vorschlag entscheidende Vorteile gegenüber bisher verwendeten Methoden erzielt werden können. Für den Patienten ist ausschlaggebend, dass die drucklos geformte Maske ein extrem geringes Gewicht hat, und der Tragekomfort entscheidend verbessert wird, zumal die erfindungsgemäße Maske in ihren äußeren Abmessungen so ausgelegt ist, dass sie im wesentlichen nur den Nasenbereich umschließt und mit einem weich bleibenden Rand versehen ist, der eine dichte Verbindung zwischen Maskenrand und Gesicht vorsieht.

Nachstehend wird die Erfindung in Verbindung mit der Zeichnung anhand eines Ausführungsbeispieles erläutert. Es zeigt:
- Fig. 1: eine schematische Darstellung des Verfahrens nach der Erfindung,
- Fig. 2: das drucklose Abformverfahren nach der Erfindung in Form eines Blockschaltbildes, und
- Fig. 3: in schematischer Darstellung die fertige Maske, die an ein Gesicht angelegt wird.

Auf das Gesicht einer liegenden Person wird zunächst unmittelbar auf die Haut Vaseline aufgetragen und anschließend vorbereitetes Abformmaterial in Form einer Abformschicht auf die abzudeckende Gesichtspartie, vorzugsweise den Nasenbereich abdeckend, aufgebracht, z.B. aufgegossen. Die Abformschicht erhält dabei eine Dicke von ca. 3 - 5 cm, wobei das Abformmaterial vorzugsweise aus Abformgips oder Silikon, welche beide rasch aushärten, besteht, so dass die Abformschicht nach dem Aushärten ein Negativ-Modell darstellt, das vom Gesicht abgenommen wird. Entscheidend ist dabei die drucklose Abformung (Herstellung) des Negativs, bei dem sich das Material in zähfließender Konsistenz von selbst der jeweiligen Form der Nasen- bzw. Gesichtspartie anpassen kann.

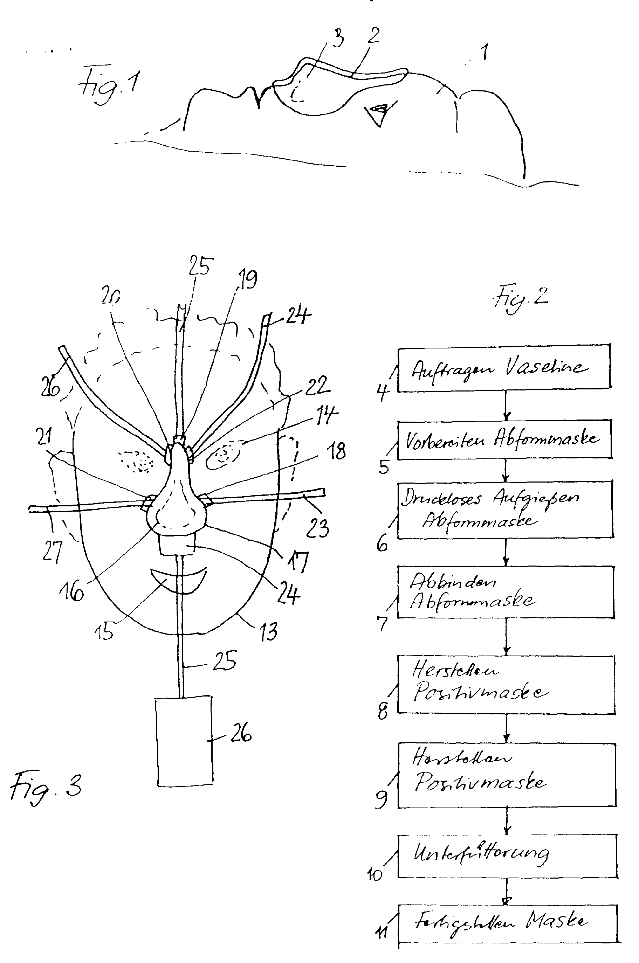

Die einzelnen Verfahrensschritte sind in Fig. 2 im Ablauf anhand eines Blockschalt-Flußbildes dargestellt. Mit 4 ist das Auftragen von Vaseline auf die Gesichtshaut angedeutet, 5 bezeichnet das Vorbereiten des Abformmaterials, das in zähfließender Konsistenz drucklos auf die Gesichtspartie aufgegossen wird (bei 6). Das Abbinden des Abformmaterials ist mit 7 bezeichnet, mit 8 das Herstellen eines Positivs als Arbeitsmodell, mit 9 das Herstellen einer Individual-Maske, mit 10 das Unterfüttern der Individual-Maske bei auftretenden Druckstellen nach Anprobe des Patienten und mit 11 die Fertigstellung der Maske durch Anbringung der Halteelemente zur Fixierung der Maske am Kopf, des Adapters und des Anschließens.

Das Gesicht 13 ist schematisch mit Augen 14 und Mund 15 sowie Nase 16 angedeutet. Im Nasenbereich wird die fertiggestellte Individual-Maske 17 aufgesetzt, die die Nasenseitenwände und Nasenflügel umschließt, nach oben bis zur Stirn reicht und nach unten oberhalb der Oberlippe endet, so dass die Nasenlöcher abdichtend umschlossen werden. Am Umfangsrand der Maske sind in zweckmäßiger Weise verteilt Bügel 18 - 22 vorgesehen, in die Haltebänder 23 - 27 eingesetzt sind, die miteinander verbindbar bzw. am Hinterkopf festlegbar sind.

An der die Nasenlöcher umschließenden Stelle der Maske ist ein Adapter 24 angeschlossen, von dem ein Schlauch 25 zu einem Beatmungsgerät 26 führt, das die Luft- oder Sauerstoff-Zufuhr steuert.

## Patentansprüche

1. Verfahren zum Abformen und Anfertigen einer nasalen Maske für die nasale Überdruck-Beatmung (nCPAP, nBIPAP), Intensivpatienten-Beatmung, intermittierende Selbstbeatmung oder dergl., **dadurch gekennzeichnet, dass**
a) auf die abzudeckende Gesichtspartie eine Trenn- bzw. Vaselineschicht aufgetragen wird,
b) selbsthärtendes Abformmaterial in Form von Abformgips oder Silikonen mit Wasser bzw. nach Herstellerangaben gemischt und zu einer zähflüssigen Masse angerührt wird,
c) die zähflüssige Abformmasse drucklos und als ca. 3 - 5 cm dicke Schicht auf die abzudeckende Gesichtspartie aufgebracht wird,
d) die abgebundene Abformung abgenommen und von dem Negativ mittels Gips ein Positiv als Arbeitsmodell hergestellt wird,
e) von dem Positiv eine individuelle nasale Beatmungsmaske hergestellt wird,
f) die empfindlichen Gesichtspartien (vor allem Nasenwurzel) je nach Bedarf des Patienten mit weichbleibendem Kunststoff oder einem Heißpolymerisat aus der Dentaltechnik unterfüttert werden.

2. Maske für die nasale Überdruck-Beatmung, Intensivpatientenbeatmung, intermittierende Selbstbeatmung oder dergl., **dadurch gekennzeichnet,**
**dass** der Maskenkörper aus einer durch drucklose Abformung gewonnenen Schicht aus Abformmaterial mit einer Dicke von 3 - 5 mm besteht,
**dass** der Maskenkörper auf den oberen Gesichtsbereich (Nasenbereich, Nasenwurzel, Stirn) beschränkt ist und der Maskenkörper auf der der Oberlippe zugewandten Seite einen genormten Adapter mit einem Anschluss für einen zu einem Beatmungsgerät führenden Druckschlauch aufweist,
**dass** am rechten und linken sowie oberen Rand des Maskenkörpers Haltebügel zur Befestigung von Haltebändern, welche zur Fixierung der Maske am Kopf dienen, integriert sind.
